**Europäisches Patentamt**

**European Patent Office**

**Office européen des brevets**

(19)

(11) Veröffentlichungsnummer: **0 205 792**
A2

# (12) EUROPÄISCHE PATENTANMELDUNG

(21) Anmeldenummer: **86104934.4**

(22) Anmeldetag: **10.04.86**

(51) Int. Cl.⁴: **G 01 N 37/00**

(30) Priorität: **15.06.85 DE 3521535**

(43) Veröffentlichungstag der Anmeldung: **30.12.86**
**Patentblatt 86/52**

(84) Benannte Vertragsstaaten: **DE FR GB**

(71) Anmelder: **Drägerwerk Aktiengesellschaft, Moislinger Allee 53-55, D-2400 Lübeck 1 (DE)**

(72) Erfinder: **Moreth, Benno, Dr. Dipl.-Phys., Marlesgrube 9, D-2400 Lübeck (DE)**
Erfinder: **Leichnitz, Kurt, Am Waldrand 42, D-2401 Gross Grönau (DE)**

(54) **Verfahren zur Kalibrierung eines Gasmessgerätes.**

(57) Ein Verfahren zur Kalibrierung eines Gasmeßgerätes, bei dem eine Meßkammer vor dem Einleiten des zu untersuchenden Gases mit einem Spülgas gespült und anschließend mit einem Kalibriergas von vorbestimmter Zusammensetzung an Kalibriersubstanz und Trägergas gefüllt wird, soll derart verbessert werden, daß das Kalibriergas nicht in seiner vorbestimmten Zusammensetzung ständig mitgeführt und bereitgestellt werden muß, und daß dadurch eine Volumenverringerung des Gasmeßgerätes erzielt wird. Dazu ist vorgesehen, daß eine Fördereinrichtung aus einem Vorratsbehälter eine von einer Steuereinheit vorgegebene Menge an Kalibriersubstanz in die Meßkammer einbringt, wonach die Kalibriersubstanz sich mit dem in der Meßkammer befindlichen Spülgas zu dem Kalibriergas vorbestimmter Zusammensetzung vermischt. Außerdem wird eine Vorrichtung zur Durchführung des Verfahrens zur Kalibrierung eines Gasmeßgerätes angegeben.

EP 0 205 792 A2

D r ä g e r w e r k  Aktiengesellschaft
Moislinger Allee 53-55, 2400 Lübeck, DE

Verfahren zur Kalibrierung eines Gasmeßgerätes

Die Erfindung betrifft ein Verfahren zur Kalibrierung
eines Gasmeßgerätes, bei dem eine Meßkammer vor dem Einleiten des zu untersuchenden Gases mit einem Spülgas gespült und anschließend mit einem Kalibriergas von vorbestimmter Zusammensetzung an Kalibriersubstanz und Trägergas gefüllt wird. Außerdem wird eine zur Durchführung des
Verfahrens geeignete Vorrichtung angegeben.
Bei Langzeit-Einsätzen von Gasmeßgeräten, welche die Gaszusammensetzung in einer Meßkammer bestimmen, ist es in
gewissen Zeitabständen immer notwendig, eine Kalibrierung
der gesamten Meßanordnung durchzuführen. Dazu wird ein
Meßgas festgesetzter Zusammensetzung aus einem Vorratsbehälter in die Meßkammer geführt. Bevor dies jedoch erfolgen kann, ist eine Spülung der Meßkammer mit einem
Spülgas, im allgemeinen Luft, notwendig, um mögliche
Restmengen vorhandenen Meßgases auszuspülen und eine Verfälschung des Kalibrierwertes zu verhindern. Zur Durchführung der Kalibrierung sind aufwendige Leitungsführungen
und Schaltanordnungen zur rechtzeitigen dosierten Zuführung von a) Meßgas, b) Spülgas (Inertgas), c) Kalibriergas notwendig. Außerdem sind Speicher für fertige
Kalibriergasgemische mitzuführen.

Ein derartiges Verfahren und eine Vorrichtung dazu wird
in der US-A-3,792,272 beschrieben.

Während des Meßzyklus wird über eine Meßleitung eine
Meßkammer mit dem zu untersuchenden Gas gefüllt und in ihr
analysiert. Wenn eine Kalibrierung der Meßkammer notwendig ist, wird über geeignete Schaltelemente eine Spülleitung mit der Meßkammer verbunden, durch welche Umgebungsluft als Spülgas in die Meßkammer eingeleitet wird. Nach durchgeführter Durchspülung der Meßkammer wird in einem weiteren Schritt eine zusätzliche Kalibriergas-Leitung an die Meßkammer angeschlossen und diese von der Spülluft-Leitung getrennt, so daß ein Kalibriergas bestimmter Zusammensetzung durch die Kalibriergas-Leitung in die Meßkammer strömt. Nach Beendigung des Kalibriervorganges wird die Kalibriergasleitung geschlossen und die Meßkammer mit der Meßgasleitung verbunden.

Bei dem bekannten Verfahren ist von Nachteil, daß der mitzuführende Kalibriergasbehälter einen großvolumigen Vorrat an fertig zusammengesetztem Kalibriergas enthalten muß. Bei einer Kalibrierung sind nicht nur die Meßkammer selbst, sondern auch die Gasleitungen zu spülen. Dadurch erhöht sich der Kalibriergasverbrauch, und eine entsprechende Vorratsmenge ist bereitzustellen. Die Herstellung des Kalibriergases vorbestimmter Zusammensetzung ist aufwendig, und dessen Bereitstellung muß außerdem in sorgfältiger Weise durchgeführt werden. So ist dabei darauf zu achten, daß sich die Zusammensetzung des Kalibriergases während der Einsatzdauer des Gasmeßgerätes nicht verändert, indem beispielsweise eine Komponente des Kalibriergases auskondensiert oder sich anderweitig in den Rohrleitungen abscheidet und nicht in die Meßkammer gelangt. Demzufolge sind unter gewissen Bedingungen dem Kalibriergas Stabilisatoren zuzusetzen, oder es ist dafür Sorge zu tragen, daß die Kalibriergasbehälter von Zeit zu Zeit ausgewechselt werden.

Der vorliegenden Erfindung liegt die Aufgabe zugrunde, ein Verfahren zur Kalibrierung eines Gasmeßgerätes derart zu verbessern, daß das Kalibriergas nicht in seiner vorbestimmten Zusammensetzung ständig mitgeführt und bereitgestellt werden muß, und daß damit eine Volumenverringerung des Gasmeßgerätes erzielt wird.

Die Lösung der genannten Aufgabe erfolgt dadurch, daß eine Fördereinrichtung aus einem Vorratsbehälter eine von einer Steuereinheit vorgegebene Menge an Kalibriersubstanz in die Meßkammer einbringt, wonach die Kalibriersubstanz sich mit dem in der Meßkammer befindlichen Spülgas zu dem Kalibriergas vorbestimmter Zusammensetzung vermischt.

Der Vorteil der Erfindung ist darin zu sehen, daß das Kalibriergas erst im Bedarfsfalle in der Meßkammer selbst erzeugt wird, wodurch eine Bevorratung der Kalibriersubstanz in ihrer stabilsten und raumsparendsten, transportgünstigsten Form möglich wird. Die Kalibrierung mit dem Kalibriergas kann in einfacher Weise ohne Zusatzaufwand an Leitungsführungen und damit unter geringstem Verbrauch an Kalibriersubstanz durchgeführt werden.

Eine Vorrichtung zur Durchführung des Verfahrens besteht aus einer Fördereinrichtung, welche über eine Förderleitung mit einem die Kalibriersubstanz enthaltenden Vorratsbehälter und über eine Zuführungsleitung mit der Meßkammer verbunden ist. Eine solche Vorrichtung bringt die für das Kalibriergas notwendige Menge an Kalibriersubstanz unmittelbar in die Meßkammer ein, in welcher sie sich mit dem dort befindlichen Spülgas zu einem Kalibriergas vorbestimmter Zusammensetzung vermischt.

Die Kalibriersubstanz in dem Vorratsbehälter kann aus einem stabilen, hochkonzentrierten Gas oder auch vorteilhafterweise aus einer Flüssigkeit bestehen, welche in die Meßkammer eingeleitet wird und in ihr verdampft.

Bei Flüssigkeiten mit hohem Dampfdruck kann auch die Dampfphase von der Fördereinrichtung in die Meßkammer eingeleitet werden.

Zur gleichmäßigen und reproduzierbaren Dosierung der Mengen an Kalibriersubstanz wird vorzugsweise eine Dosierpumpe eingesetzt.

Zeitpunkt und die Zeitdauer einer Dosierung der Kalibriersubstanz wird von einer Steuereinheit vorgegeben.

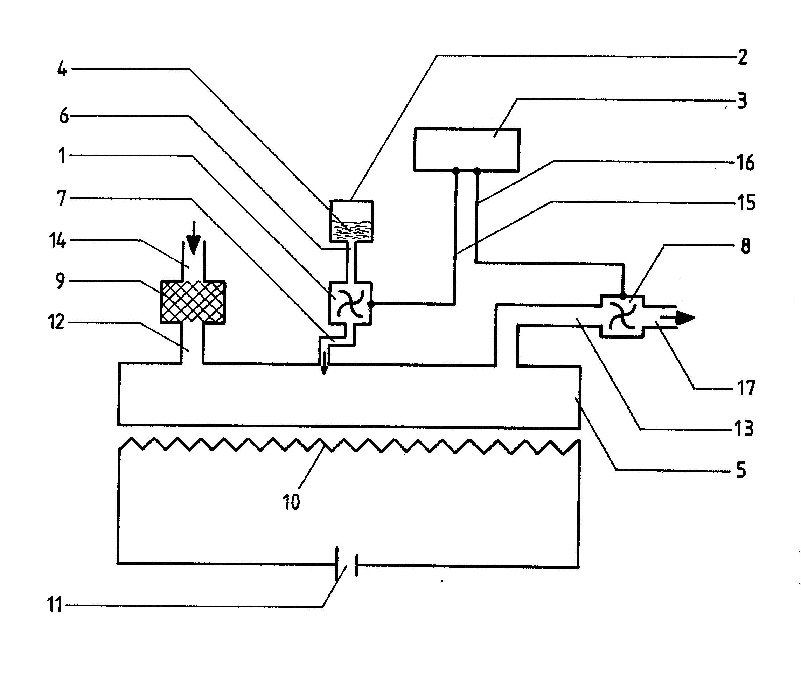

Das Ausführungsbeispiel einer Vorrichtung zur Durchführung des Verfahrens zur Kalibrierung eines Gasmeßgerätes wird anhand der einzigen Figur schematisch dargestellt und im folgenden näher erläutert.

Das Blockschaltbild der Vorrichtung zeigt eine Meßkammer 5, welche einen Gaseinlaßkanal 12 und einen Gasauslaßkanal 13 besitzt. Darüberhinaus ist sie mit einer Zuführungsleitung 7 verbunden, über welche mittels einer als Dosierpumpe 1 ausgebildeten Fördereinrichtung aus dem Vorratsbehälter 2 die Kalibriersubstanz 4 durch die Förderleitung 6 in die Meßkammer 5 eingeführt wird. An den Gasauslaßkanal 13 ist eine Förderpumpe 8 angeschlossen, an der sich eine Ausströmleitung 17 befindet. Dosierpumpe 1 und Förderpumpe 8 sind über entsprechende Steuerleitungen 15, 16 mit einer Steuereinheit 3 verbunden. Die Meßkammer 5 ist zur Erzeugung stabiler Temperaturbedingungen mit einer Heizung 10 versehen, welche über eine Spannungsquelle 11 mit Energie versorgt wird.

Zur Durchführung des Kalibriervorganges wird zunächst über die Steuereinheit 3 und die Steuerleitung 16 die Förderpumpe 8 betrieben, wodurch der Innenraum der Meßkammer 5 mit der durch den Anschlußstutzen 14 und das Filter 9 und den Gaseinlaßkanal 12 einströmenden Spülluftmenge bespült wird. Nach einer vorgebbaren Zeitspanne wird die Förderpumpe 8 abgeschaltet und die Dosierpumpe 1 durch die Steuereinheit 3 über die Steuerleitung 15 eingeschaltet. Daraufhin wird eine durch die von der Steuereinheit 3 vorgegebene Einschaltdauer bestimmte Menge an Kalibriersubstanz 4 aus dem Vorratsbehälter 2 durch die Förderleitung 6 und die Zuführungsleitung 7 in die Meßkammer transportiert. Dort vermischt sie sich mit der in der Meßkammer 5 befindlichen Spülluft zu einem Kalibriergas vorbestimmter Zusammensetzung, welche durch das Verhältnis der Anteile an Spülgas und Kalibriersubstanz festgelegt ist. Die Vermischung von Kalibriersubstanz 4 und Spülluft wird durch die von der Heizung 10 verursachte Konvektion innerhalb der Meßkammer 5 noch begünstigt.

Patentansprüche

1. Verfahren zur Kalibrierung eines Gasmeßgerätes, bei dem eine Meßkammer vor dem Einleiten des zu untersuchenden Gases mit einem Spülgas gespült und anschließend mit einem Kalibriergas von vorbestimmter Zusammensetzung an Kalibriersubstanz und Trägergas gefüllt wird, d a d u r c h  g e k e n n z e i c h n e t, daß eine Fördereinrichtung (1) aus einem Vorratsbehälter (2) eine von einer Steuereinheit (3) vorgegebene Menge an Kalibriersubstanz (4) in die Meßkammer (5) einbringt, wonach die Kalibriersubstanz (4) sich mit dem in der Meßkammer (5) befindlichen Spülgas zu dem Kalibriergas vorbestimmter Zusammensetzung vermischt.

2. Vorrichtung zur Durchführung des Verfahrens nach Anspruch 1, d a d u r c h  g e k e n n z e i c h n e t, daß eine Fördereinrichtung (1) über eine Förderleitung (6) mit einem die Kalibriersubstanz (4) enthaltenden Vorratsbehälter (2) und über eine Zuführungsleitung (7) mit der Meßkammer (5) verbunden ist.

3. Vorrichtung nach Anspruch 2, d a d u r c h  g e k e n n z e i c h n e t, daß der Vorratsbehälter (2) mit einer flüssigen Kalibriersubstanz (4) gefüllt ist.

4. Vorrichtung nach Anspruch 2, d a d u r c h  g e k e n n z e i c h n e t, daß die Fördereinrichtung als Dosierpumpe (1) ausgebildet ist.

5. Vorrichtung nach Anspruch 2, d a d u r c h  g e k e n n z e i c h n e t, daß die Fördereinrichtung (1) zur Abgabe vorbestimmter Mengen an Kalibriersubstanz (4) mit einer Steuereinheit (3) verbunden ist.